# EUROPEAN PATENT APPLICATION

(11) **EP 4 086 336 A1**
(43) Date of publication of application: **09.11.2022**
(21) Application number: 20910843.0
(22) Date of filing: 29.12.2020
(51) Int. Cl.: C12N 1/20, A61K 35/747, A61K 47/10, A61K 47/26, A61K 47/36, A61P 3/04, A23K 10/16, A23L 33/135

(54) **LACTOBACILLUS PLANTARUM STRAIN, AND COMPOSITION FOR PREVENTING OR TREATING METABOLIC DISEASES CONTAINING SAME**

(30) Priority: 31.12.2019 KR 20190178951
(71) Applicant: GI BIOME, Gyeonggi-do 13201 (KR)
(72) Inventor: YANG, Bo Gie, Seoul 05849 (KR); JANG, Myung Ho, Seoul 05849 (KR); KANG, Chang Ho, Chungju-si Chungcheongbuk-do 27373 (KR); PAEK, Nam Soo, Seoul 06608 (KR)
(74) Representative: HGF
(86) International application number: PCT/KR2020/019347
(87) International publication number: WO 2021/137603

(57) **Abstract**

Provided is a novel *Lactobacillus Plantarum* strain and a composition for preventing or treating a metabolic disease including the same. A *Lactobacillus Plantarum* (Accession No. KCTC 14107BP) according to the present invention may inhibit fat accumulation in white adipose tissue, brown adipose tissue, and liver tissue. The *Lactobacillus Plantarum* strain may exhibit excellent blood glucose improvement effect, and in particular, may reduce a fasting blood glucose level. The *Lactobacillus Plantarum* strain may effectively ameliorate insulin resistance by improving glucose tolerance and increasing insulin sensitivity. In addition, the *Lactobacillus Plantarum* strain may regulate a concentration of metabolic hormones in blood. Therefore, the *Lactobacillus Plantarum* strain may be usefully used to prevent or treat a metabolic disease.

## Description

### Technical Field

The present disclosure relates to a novel *Lactobacillus Plantarum* strain and a composition for preventing or treating a metabolic disease including the same.

### Background Art

As society develops, obesity has emerged as one of the serious diseases, and the World Health Organization (WHO) has recognized obesity as a disease to be treated. Recently, the trend toward increasing prevalence of obesity is also observed in Republic of Korea due to westernized diet, and there is a growing interest in treatment and prevention therefor. Obesity refers to a state of an excessive accumulation of body fat resulting from an imbalance between food consumption and energy expenditure. In addition, obesity is closely related to insulin resistance, glucose tolerance, hyperlipidemia, etc., and may be accompanied by metabolic diseases including cardiovascular diseases, fatty liver diseases, cancers, and diabetes as complications.

Recently, as it became known that the inflammatory response in adipose tissue of an obese patient is increased, obesity is sometimes considered as a low-grade systemic inflammation. In particular, it has been reported that inflammatory response is increased by inflammatory macrophages that are increased in proportion to the size of adipose tissue, inflammatory adipokines produced and secreted by adipose tissue in such a state is a pathogenic factor of metabolic diseases such as cardiovascular diseases and diabetes. Therefore, obesity which increases these molecules can be seen as a cause of almost all adult diseases.

Currently prescribed obesity medicines are Xenical (Roche Pharmaceuticals., Switzerland), Reductil (Abbott Co., US) and Exolise (Arkopharma LLC, France), or the like. The obesity medicines are largely classified as appetite suppressants, energy expenditure promoters, or fat absorption inhibitors, and most of the obesity medicines are appetite suppressants that suppress appetite by controlling neurotransmitters related to hypothalamus. However, conventional obesity medicines have side effects such as heart diseases, respiratory diseases, and nervous system diseases, and also have a problem in that in vivo persistence thereof is low. Accordingly, there is a need to develop safe and effective obesity medicines.

Meanwhile, probiotics to prevent or treat obesity using safe microorganisms (e.g., lactic acid bacteria) has been actively studied. In particular, research has shown that lactic acid bacteria exhibit effects such as maintenance of normal intestinal flora, improvement of intestinal flora, antidiabetic and antilipidemic effects, inhibition of carcinogenesis, inhibition of colon cancer, and nonspecific activity of the host's immune system.

Regarding lactic acid bacteria related to obesity prevention and treatment effect, KR Patent No. 10-1494279 discloses a *Lactobacillus plantarum* KY1032 strain (Accession No. KCCM-10430) having an inhibitory effect on adipocyte differentiation, KR Patent No. 10-0996577 discloses a *Lactobacillus curvatus* HY7601 (Accession No. KCTC 11456BP) having obesity inhibitory effect, and KR Patent No. 10-1394348 discloses a *Lactobacillus plantarum* DSR920 strain (Accession No. KCCM 11210P) having an inhibitory effect on adipocyte differentiation, but none of them is mature enough to obtain commercial success.

Therefore, there is a need to continue research on a new strain having excellent anti-obesity effect.

### Detailed Description of the Invention

### Technical Problem

Accordingly, the present inventors found out that a *Lactobacillus Plantarum* strain (Accession No. KCTC 14107BP) exhibits excellent anti-obesity effect as a result of researching to develop a new strain with excellent anti-obesity effect, and thereby have completed the present invention.

### Solution to Problem

An aspect of the present invention provides a *Lactobacillus Plantarum* strain (Accession No. KCTC 14107BP).

Another aspect of the present invention provides a pharmaceutical composition for preventing or treating a metabolic disease including, as an active ingredient, a *Lactobacillus Plantarum strain* (Accession No. KCTC 14107BP) or a culture thereof.

Yet another aspect of the present invention provides a food composition for preventing or inhibiting a metabolic disease including a *Lactobacillus Plantarum* strain (Accession No. KCTC 14107BP) or a culture thereof.

Still another aspect of the present invention provides a feed composition for preventing or inhibiting a metabolic disease including a *Lactobacillus Plantarum* strain (Accession No. KCTC 14107BP) or a culture thereof.

Yet still another aspect of the present invention provides a method of preventing or treating a metabolic disease including a *Lactobacillus Plantarum* strain (Accession No. KCTC 14107BP) or a culture thereof.

### Effect of the Invention

The present disclosure relates to a *Lactobacillus Plantarum* strain and a composition for preventing or treating a metabolic disease including the same. *Lactobacillus Plantarum* (Accession No. KCTC 14105BP) according to the present invention may effectively inhibit fat accumulation in white adipose tissue, brown adipose tissue, and liver tissue. The *Lactobacillus Plantarum* strain may exhibit excellent blood glucose improvement effect, and in particular, may reduce a fasting blood glucose level. The *Lactobacillus Plantarum* strain may effectively ameliorate insulin resistance by improving glucose tolerance and increasing insulin sensitivity. In addition, the *Lactobacillus Plantarum* strain may regulate the concentration of metabolic hormones in blood. Therefore, *the Lactobacillus Plantarum* strain may be usefully used to prevent or treat a metabolic disease.

### Brief Description of Drawings

Exemplary embodiments can be understood in more detail from the following description taken in conjunction with the accompanying drawings, in which:

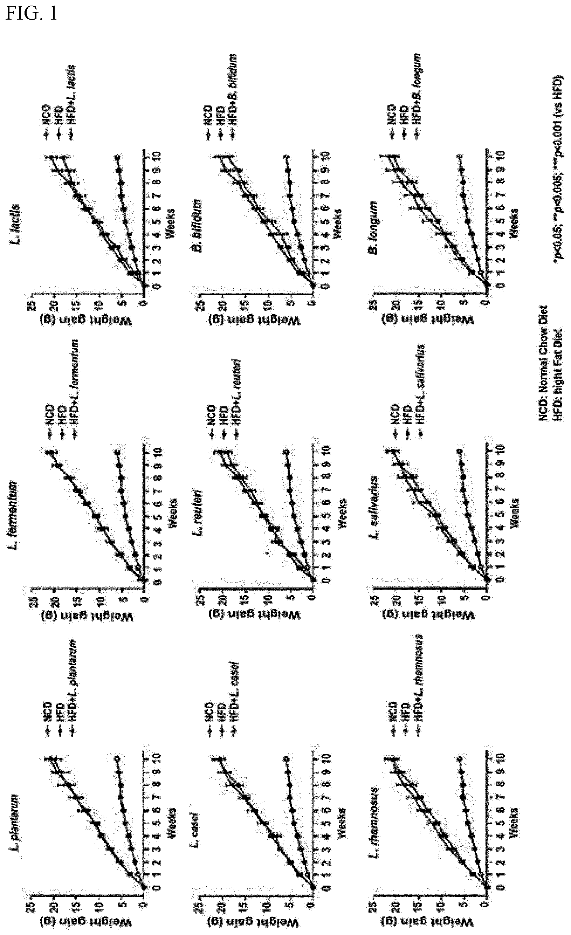

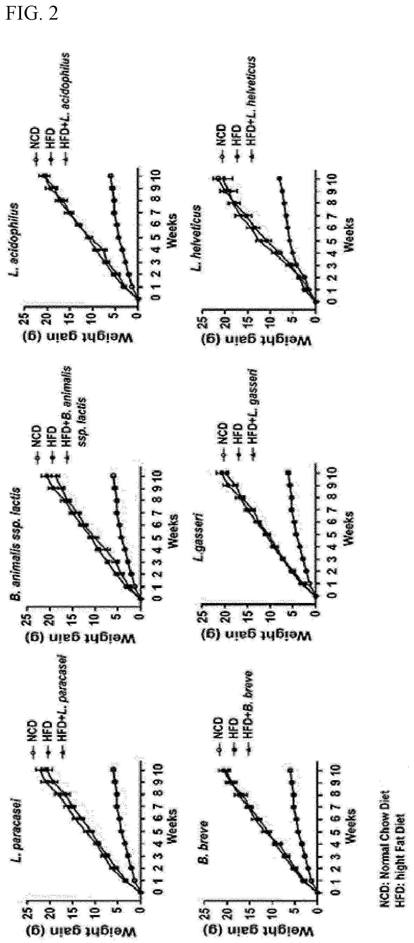

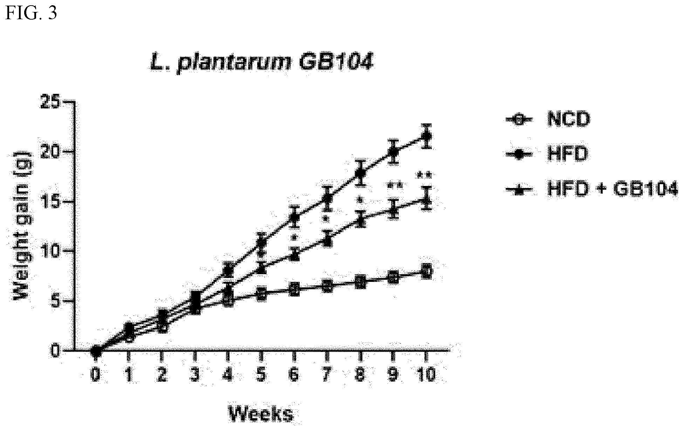

FIGS. 1 to 3 are graphs showing the body weight of the mice fed with high fat diet after oral administration of 16 strains of lactic acid bacteria, respectively, compared to the mice only fed with high fat diet;

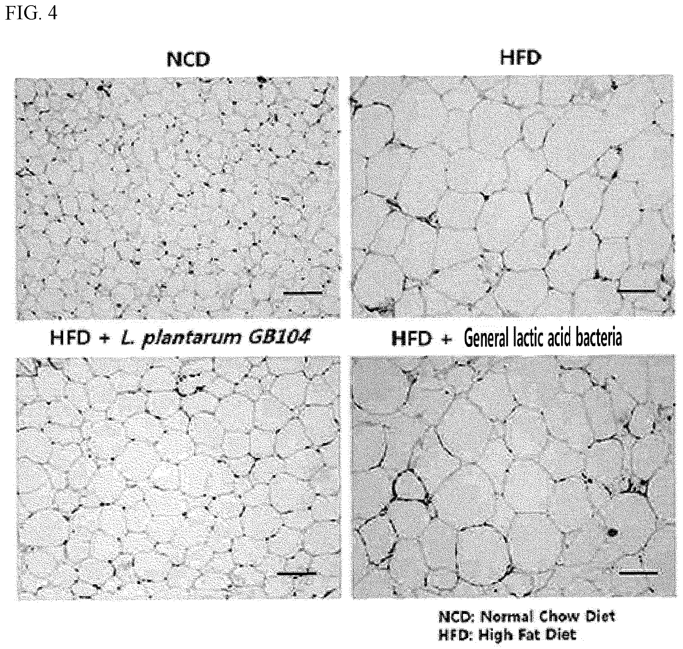

FIG. 4 is images comparing the size of the adipocytes in white adipose tissue each from the mouse fed with normal chow diet, the mouse fed with high fat diet, the mouse fed with high fat diet and oral administration of general lactic acid bacteria, and the mouse fed with high fat diet and an oral administration of *L. Plantarum GB104* strain to compare;

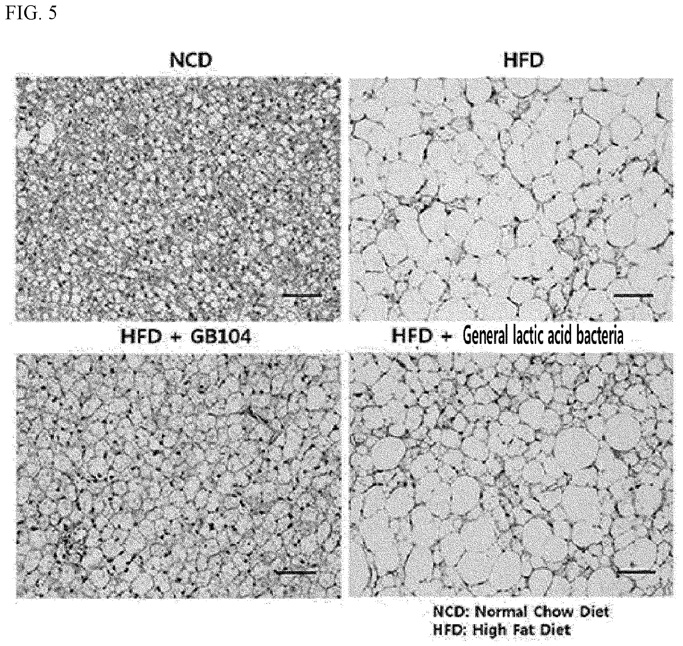

FIG. 5 is images comparing the size of the adipocytes in brown adipose tissue of the mouse fed with normal chow diet, the mouse fed with high fat diet, the mouse fed with high fat diet and oral administration of general lactic acid bacteria, and the mouse fed with high fat diet and oral administration of a *L*. *Plantarum GB104* strain;

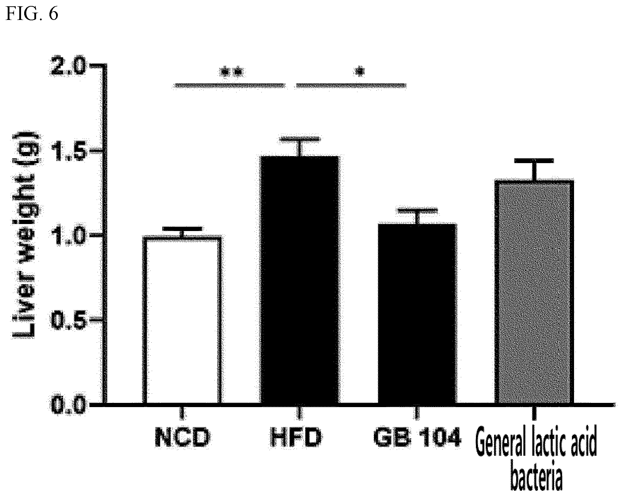

FIG. 6 is a graph comparing the liver weight of the mouse fed with normal chow diet, the mouse fed with high fat diet, the mouse fed with high fat diet and oral administration of general lactic acid bacteria, and the mouse fed with high fat diet and an oral administration of a *L. Plantarum GB104* strain;

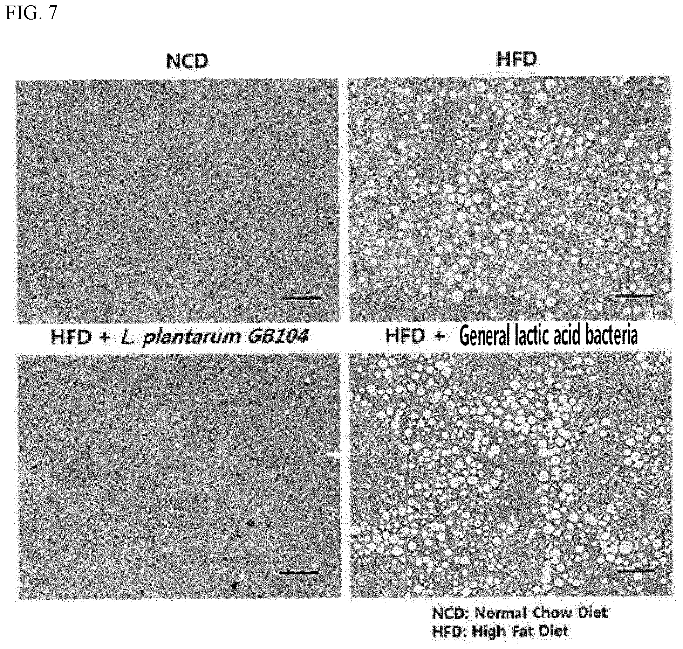

FIG. 7 is images comparing the degree of fat accumulation in liver tissue of the mouse fed with normal chow diet, the mouse fed with high fat diet, the mouse fed with high fat diet and oral administration of general lactic acid bacteria, and the mouse fed with high fat diet and an oral administration of a *L*. *Plantarum GB104;*

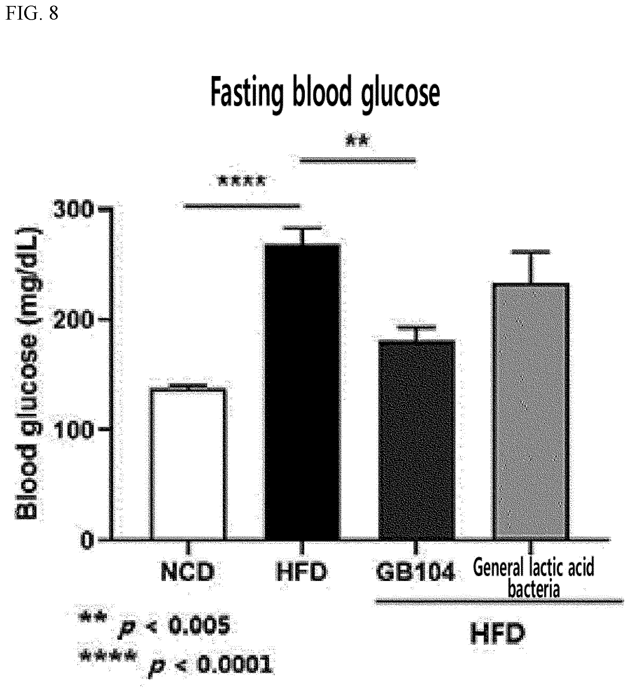

FIG. 8 is a graph comparing the fasting blood glucose level of the mouse fed with normal chow diet, the mouse fed with high fat diet, the mouse fed with high fat diet and oral administration of general lactic acid bacteria, and the mouse fed with high fat diet and an oral administration of a *L. Plantarum GB104* strain to confirm blood glucose lowering effect;

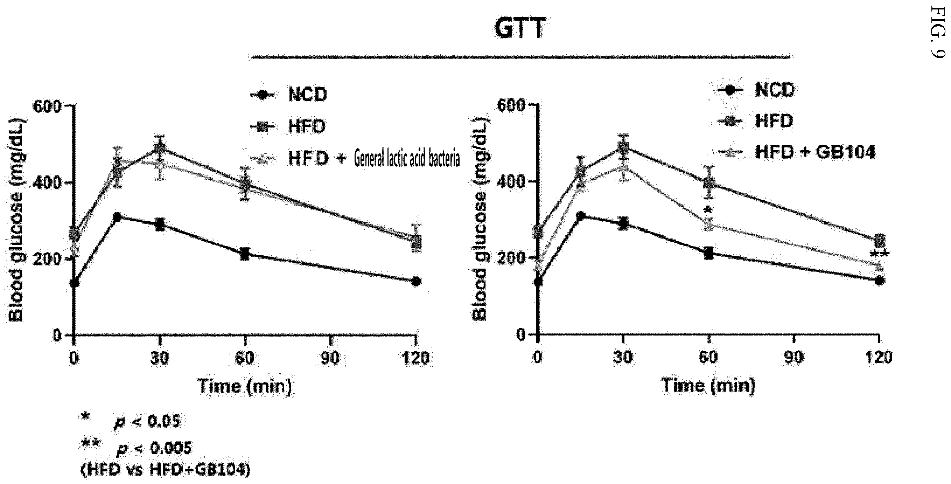

FIG. 9 is a graph comparing the blood glucose level after administration of glucose over time in the mouse fed with normal chow diet, the mouse fed with high fat diet, the mouse fed with high fat diet and oral administration of general lactic acid bacteria, and the mouse fed with high fat diet and an oral administration of a *L. Plantarum GB104* strain to confirm glucose tolerance improvement effect;

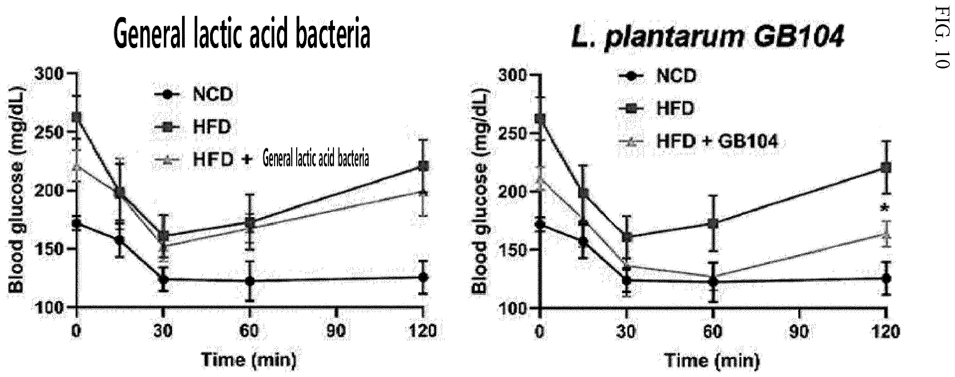

FIG. 10 is a graph comparing the blood glucose level after administration of insulin over time in the mouse fed with normal chow diet, the mouse fed with high fat diet, the mouse fed with high fat diet and oral administration of general lactic acid bacteria, and the mouse fed with high fat diet and an oral administration of a *L. Plantarum* GB104 strain to confirm insulin resistance ameliorating effect; and

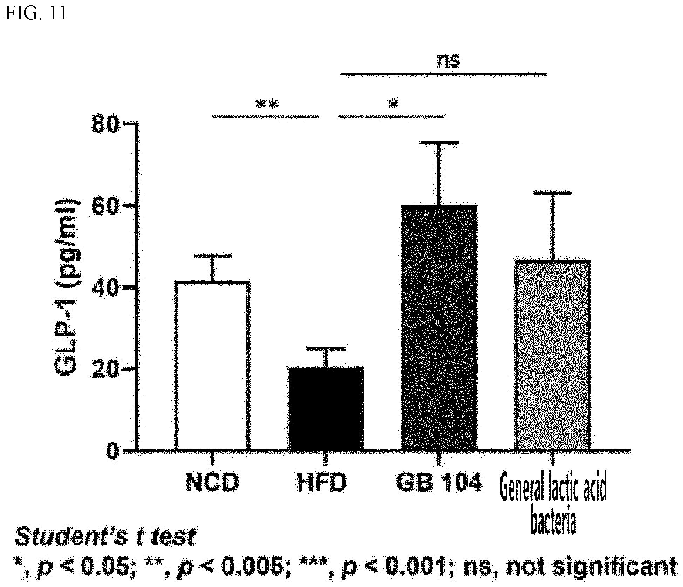

FIG. 11 is a graph comparing the concentration of glucagone-liked peptide-1 (GLP-1) in blood of the mouse fed with normal chow diet, the mouse fed with high fat diet, the mouse fed with high fat diet and oral administration of general lactic acid bacteria, and the mouse fed with high fat diet and an oral administration of a *L. Plantarum GB104* strain to confirm the ability to regulate metabolic hormones.

### Best Mode for Carrying out the Invention

An aspect of the present invention provides a *Lactobacillus Plantarum* strain (Accession No. KCTC 14107BP).

*Lactobacillus* is an aerobic or facultative anaerobic, gram-positive bacillus widely distributed in nature. Genus *Lactobacillus* includes *L. Plantarum, L.* sakei, etc. The present inventors selected a novel *Lactobacillus Plantarum* strain having excellent anti-obesity effect, and named it *"Lactobacillus Plantarum GB104."* The strain was deposited with the Korea Collection for Type Cultures, Korea Research Institute of Bioscience and Biotechnology under the Accession No. SD1337 on September 6, 2019. The same strain was deposited with the Korea Collection for Type Cultures, Korea Research Institute of Bioscience and Biotechnology under the Accession No. KCTC 14107BP on January 14, 2020. In addition, the strain belongs to a probiotic strain, is harmless to the human body, and may be used without side effects.

As used herein, the term *"Lactobacillus Plantarum GB104"* is interchangeably described as *L. Plantarum GB104* or *Lactobacillus Plantarum* strain (Accession No. KCTC 14107BP).

Another aspect of the present invention provides a pharmaceutical composition for preventing or treating a metabolic disease including, as an active ingredient, a *Lactobacillus Plantarum strain* (Accession No. KCTC 14107BP) or a culture thereof.

The *Lactobacillus Plantarum* strain (Accession No. KCTC 14107BP) is the same as described above. In this case, the strain may be alive or dead, and alive strain is preferred. In addition, a culture of the strain may or may not contain the strain, and it is preferred to contain the strain.

The composition includes, based on the total weight of the composition, a therapeutically effective amount, or a nutritionally effective concentration of the active ingredient *Lactobacillus Plantarum* strain (Accession No. KCTC 14107BP) or a culture thereof, wherein a content of 10⁴ to 10¹⁶ CFU/g, preferably 10⁶ to 10¹² CFU/g is included, or a culture having an equivalent number of alive strains is included. In general, for an adult patient, 1×10⁶ CFU/g or more of the alive strain, preferably 1×10⁸ to 1×10¹² CFU/g of the alive strain may be administered once or divided in several times.

As used herein, the term "metabolic disease", also referred to as metabolic syndrome, is a disease assumed to be caused by insulin resistance and is a symptom having abnormality in two or more of cholesterol, blood pressure, and blood glucose levels. Metabolic syndrome is a conceptualization of the phenomenon that the risk factors of various cardiovascular diseases and type 2 diabetes are clustered together as one disease group, and encompasses insulin resistance and all of complex and various metabolic abnormalities and clinical features related to insulin resistance. Specifically, metabolic disease may be any one selected from the group consisting of obesity, hypertension, arteriosclerosis, hyperlipidemia, fatty liver, non-alcoholic fatty liver disease, hyperinsulinemia, diabetes, and insulin resistance syndrome.

As used herein, the term "obesity", also referred to as adipositas, is a disease in which excess body fat has accumulated abnormally. Irregular eating habits, excessive food intake, lack of physical activity, endocrine system diseases, genetic factors, psychological factors, medication, etc. can cause obesity. In addition, obesity increases the incidence of arteriosclerosis, cardiovascular diseases (stroke and ischemic cardiovascular diseases), hypertension, diabetes, hyperlipidemia, fatty liver, cholelithiasis, obstructive sleep apnea, menstrual irregularities, polycystic ovary diseases, infertility, decreased libido, depression, degenerative arthritis, gout, or the like. The obesity may be simple obesity, symptomatic obesity, childhood obesity, adult obesity, cell proliferative obesity, cell hypertrophic obesity, upper body obesity, lower body obesity, visceral fat obesity, or subcutaneous fat obesity.

As used herein, the term "hypertension" refers to a phenomenon in which perfusion blood pressure of blood flow through an artery increases. If the systolic blood pressure reaches 140 mmHg and the diastolic blood pressure reaches 90 mmHg or more, one may generally be diagnosed with hypertension. Hypertension has no noticeable symptoms, and there are primary (or essential) hypertension which does not have a known cause and secondary hypertension which is caused by renal disease, endocrine disease, pregnancy addiction, or the like. Most cases of hypertension (90 to 95%) are primary hypertension assumed to be caused by genetic causes combined with environmental factors such as obesity, stress, alcohol, and smoking.

As used herein, the term "arteriosclerosis" is defined as a phenomenon of narrowing the width of the arterial wall by loss of the elasticity of the walls of arteries, proliferation of abnormal tissue, and accumulation of fat inside the lining of the artery wall. Arteriosclerosis is a term that refers to a pathological change in an artery, and is named depending on the organs affected by arteriosclerosis. For example, arteriosclerosis includes, but is not limited to, cerebral infarction due to arteriosclerosis, and myocardial infarction due to coronary atherosclerosis.

As used herein, the term "hyperlipidemia" is a disease caused by large amount of lipids such as triglycerides and cholesterol in blood because lipid metabolism is not properly performed. Specifically, hyperlipidemia refers to a state in which lipid components such as triglycerides, LDL cholesterol, and free fatty acids in blood are increased. Hyperlipidemia includes, but is not limited to, hypercholesterolemia or hypertriglyceridemia.

As used herein, the term "fatty liver" is a state of excessive accumulation of fat in hepatocytes due to lipid metabolism disorder, and is defined as a case when fat reaches 5% or more of the liver weight. This causes various diseases such as angina pectoris, myocardial infarction, stroke, arteriosclerosis, fatty liver, pancreatitis, or the like. Fatty liver is divided into alcoholic fatty liver that is caused by alcohol consumption, and non-alcoholic fatty liver disease (NAFLD) that is not caused by alcohol.

As used herein, the term "non-alcoholic fatty liver disease" refers to a case when fatty liver is not caused by alcohol, and includes a series of process of liver damage ranging from simple steatosis in liver, non-alcoholic steatohepatitis (NASH) to hepatic cirrhosis. The causes of non-alcoholic fatty liver disease are side effects of drugs such as antiarrhythmic agents, antiviral agents, steroids, or cytotoxic agents, excessive calorie consumption such as carbohydrates, obesity, diabetes, and some genetic causes.

As used herein, the term "diabetes" is a chronic disease characterized by relative or absolute lack of insulin resulting in glucose-intolerance. The diabetes includes all types of diabetes, and may be, for example, type 1 diabetes, type 2 diabetes, and inherited diabetes, but is not limited thereto. Type 1 diabetes is insulin-dependent diabetes, mainly resulting from destruction of β-cells. As used herein, the term "type 2 diabetes" is insulin-independent diabetes, which is caused by insulin resistance. Type 2 diabetes is caused because increase in insulin is not detected in muscle and adipose tissue, or the action of insulin does not occur effectively even if it is detected.

As used herein, the term "insulin resistance" refers to a state in which cells do not respond to insulin which lower a blood glucose level, and thus cannot effectively burn glucose. When insulin resistance is high, the body thinks more insulin is needed and produces more insulin. This results in hyperinsulinemia, hypertension, or dyslipidemia, as well as heart disease and diabetes.

As used herein, the term "insulin resistance syndrome" is a generic term for the diseases caused by insulin resistance. This is characterized by cell resistance to insulin action, hyperinsulinemia and increased very low-density lipoprotein (VLDL) and triglyceride, and decreased high density lipoprotein (HDL), hypertension, etc., and is recognized as a risk factor for cardiovascular diseases and type 2 diabetes.

According to an embodiment of the present invention, the strain may inhibit fat accumulation in white adipose tissue. Specifically, in an embodiment of the present invention, oral administration of the strain to the mouse model fed with high fat diet resulted in that the size of the adipocytes in white adipose tissue of the mouse model was significantly decreased. Meanwhile, when general lactic acid bacteria were orally administered to the mouse model fed with high fat diet, the size of the adipocytes in white adipose tissue of the mouse model was not decreased. Based on this, it was confirmed that the strain inhibits fat accumulation in white adipose tissue (FIG. 4).

According to an embodiment of the present invention, the strain may inhibit fat accumulation in brown adipose tissue. Specifically, in an embodiment of the present invention, oral administration of the strain to the mouse model fed with high fat diet resulted in that the size of the adipocytes in brown adipose tissue of the mouse model was significantly decreased. Meanwhile, when general lactic acid bacteria were orally administered to the mouse model fed with high fat diet, the size of the adipocytes in brown adipose tissue of the mouse model was not decreased. Based on this, it was confirmed that the strain inhibits fat accumulation in brown adipose tissue (FIG. 5).

According to an embodiment of the present invention, the strain may reduce liver weight and inhibit fat accumulation in liver tissue. Specifically, in an embodiment of the present invention, oral administration of the strain to the mouse model fed with high fat diet resulted in that the liver weight and fat accumulation in tissue of the mouse model were decreased. Meanwhile, when general lactic acid bacteria were orally administered to the mouse model fed with high fat diet, a lot of fat was accumulated in liver tissue of the mouse model and there was no change in weight as well. Based on this, it was confirmed that the strain reduces the liver weight and inhibits fat accumulation in tissue (FIGS. 6 and 7).

According to an embodiment of the present invention, the strain may reduce a blood glucose level. Preferably, the strain may reduce a fasting blood glucose level and improve glucose tolerance. Specifically, in an embodiment of the present invention, oral administration of the strain to the mouse model fed with high fat diet resulted in that a fasting blood glucose level and a blood glucose level after administration of glucose were decreased in the mouse model. Meanwhile, when general lactic acid bacteria were orally administered to the mouse model fed with high fat diet, a fasting blood glucose level and a blood glucose level after administration of glucose were not decreased. Based on this, it was confirmed that the strain reduces a fasting blood glucose level and improves glucose tolerance (FIGS. 8 and 9).

According to an embodiment of the present invention, the strain may ameliorate insulin resistance. Specifically, in an embodiment of the present invention, when the strain was orally administered to the mouse model fed with high fat diet, the blood glucose level was significantly decreased in the mouse model after administration of insulin. Meanwhile, when general lactic acid bacteria were orally administered to the mouse model fed with high fat diet, the blood glucose level after administration of insulin were not decreased. Based on this, it was confirmed that the strain ameliorates insulin resistance by increasing insulin sensitivity (FIG. 10).

According to an embodiment of the present invention, the strain may regulate the secretion of metabolic hormones GLP-1.

As used herein, the term "metabolic hormone" refers to a hormone involved in metabolic regulation, and may preferably be incretin. As used herein, the term "incretin" is a hormone that is secreted in the gastrointestinal tract due to nutrients of food after eating food, and serves to promote the secretion of insulin in a blood glucose-dependent way in the pancreas. Incretins include GLP-1 and glucose-dependent insulinotropic peptide (GIP). The "GLP-1" acts on the pancreas to increase insulin secretion and decrease glucagon secretion, thereby exhibiting blood glucose lowering effect. In addition, GLP-1 delays gastric emptying, suppresses appetite, and improves the function of β-cells to exhibit anti-diabetic and anti-obesity effects.

In an embodiment of the present invention, oral administration of the strain to the mouse model fed with high fat diet resulted in an increase in the concentration of GLP-1 in blood which was decreased in the mouse model, but when general lactic acid bacteria were orally administered, change in the concentration of GLP-1 in blood was not observed. Based on this, it was confirmed that the strain regulates the concentration of GLP-1 in blood (FIG. 11).

The composition may further include a cryoprotectant or an excipient. Specifically, the cryoprotectant may be one or more selected from the group consisting of glycerol, trehalose, maltodextrin, skim milk powder, and starch. In addition, the excipient may be one or more selected from the group consisting of glucose, dextrin, and skim milk powder.

The composition may include, based on the total weight of the composition, 0.01 wt% to 20 wt% or 0.01 wt% to 10 wt% of the cryoprotectant, and specifically, the composition may include, 5 wt% to 20 wt% of the glycerol, 2 wt% to 10 wt% of the trehalose, 2 wt% to 10 wt% of the maltodextrin, 0.5 wt% to 2 wt% of the skim milk powder, and 0.1 wt% to 1 wt% of the starch. In addition, the composition may include, based on the total weight of the composition, 75 wt% to 95 wt% or 85 wt% to 95 wt% of the excipient.

Yet another aspect of the present invention provides a food composition for preventing or inhibiting a metabolic disease including a *Lactobacillus Plantarum* strain (Accession No. KCTC 14107BP) or a culture thereof.

The *Lactobacillus Plantarum* strain (Accession No. KCTC 14107BP) is the same as described above.

The food composition includes all forms such as functional food, nutritional supplement, health food, and food additives, and the types of food composition may be prepared in various forms according to a conventional method known in the art.

When the strain is used as a food additive, the strain may be added as it is or may be used with other food or food ingredients, and may be appropriately used according to a conventional method. The mix amount of the active ingredient may be appropriately determined depending on the purpose of use (prevention, health, or therapeutic treatment). In general, when prepare food or beverage, the active ingredient may be added in an amount of 0.0001 wt% to 1 wt%, specifically 0.001 wt% to 0.1 wt% in the raw material composition containing the strain. However, in the case of long-term intake for health and hygiene or health control purposes, the amount may be below the above range.

Yet another aspect of the present invention provides a feed composition for preventing or inhibiting a metabolic disease including a *Lactobacillus Plantarum* strain (Accession No. KCTC 14107BP) or a culture thereof.

The *Lactobacillus Plantarum* strain (Accession No. KCTC 14107BP) is the same as described above.

The feed composition for preventing or ameliorating a metabolic disease may be prepared by adding a *Lactobacillus Plantarum* strain (Accession No. KCTC 14107BP) in an appropriate effective concentration range according to various methods for preparing a feed composition known in the art.

Yet still another aspect of the present invention provides a method of preventing and treating a metabolic disease including administering a *Lactobacillus Plantarum* strain (Accession No. KCTC 14107BP) or a culture thereof to a subject.

The subject may have a metabolic disease. In addition, the subject may be a mammal, preferably a human. In this case, the *Lactobacillus Plantarum* strain (Accession No. KCTC 14107BP) is the same as described above. In addition, the route of administration, dosage, and frequency of administration of the strain or a culture thereof may be administered to a subject in various ways and amounts depending on the condition of a patient, and the presence or absence of side effects, and optimal method of administration, dosage and frequency of administration may be appropriately selected within an appropriate range by a person skilled in the art. In addition, the types of metabolic diseases are as described above.

Yet another aspect of the present invention provides a use of a *Lactobacillus Plantarum* strain (Accession No. KCTC 14107BP) or a culture thereof to treat a metabolic disease.

In this case, the *Lactobacillus Plantarum* strain (Accession No. KCTC 14107BP) is the same as described above. In addition, the types of metabolic diseases are as described above.

### Mode for Carrying out the Invention

Hereinafter, the present invention will be described in more detail by way of the following examples. However, the following examples are only for illustrating the present invention, and the scope of the present invention is not limited thereto.

### Example 1. Screening for a strain having anti-obesity effect

Lactic acid bacteria having anti-obesity efficacy among 16 lactic acid bacteria purchased from Mediogen Co., were selected using a mouse model. Specifically, C57BL/6 which were fed with 60% high fat diet (HFD), and then administered orally with the respective lactic acid bacteria, were used as an experimental group. Mice only fed with 60% high fat diet were used as a control group. In this case, each of lactic acid bacteria was orally administered at 5×10⁹ CFU per mouse daily. The anti-obesity effect was determined based on the body weight differences between the experiment group and the control group.

As a result, the *Lactobacillus Plantarum GB104* strain effectively inhibited body weight gain. In this case, there were some of *L. Plantarum* lactic acid bacteria that did not exhibit anti-obesity effect (FIGS. 1 to 3). Based on this, it was confirmed that the *Lactobacillus Plantarum GB104* strain exhibited anti-obesity effect among *L. Plantarum* lactic acid bacteria.

### Example 2. Confirmation of fat accumulation inhibitory effect of L. Plantarum GB104 strain in white adipose tissue

Using a mouse model, fat accumulation inhibitory effect of a *L. Plantarum GB104* (Accession No. KCTC 14107BP) strain in white adipose tissue was confirmed. Specifically, C57BL/6 mice which were fed with 60% high fat diet (HFD), and then administered orally with the *L. Plantarum GB104* strain, were was used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or a *L. Plantarum GB104* strain was orally administered daily at 5×10⁹ CFU per mouse.

After the administration of each test substance was completed, white adipose tissue was separated by autopsy. The separated white adipose tissue was fixed with 10% neutral buffered formalin. Then, paraffin sections were prepared, stained with Hematoxylin & Eosin (H&E), and then the size of the cells was observed.

The result showed that the size of the adipocytes in the negative and positive control groups fed with high fat diet was increased compared to the normal group, while the size of the adipocytes in the experimental group to which a *L. Plantarum GB104* strain was orally administered was significantly decreased (FIG. 4). Based on this, it was confirmed that the *L. Plantarum GB104* strain effectively inhibits fat accumulation in white adipose tissue.

### Example 3. Confirmation of fat accumulation inhibitory effect of L. Plantarum GB104 strain in brown adipose tissue

Using a mouse model, fat accumulation inhibitory effect of a *L. Plantarum GB104* strain in brown adipose tissue was confirmed. Specifically, C57BL/6 mice which were fed with 60% (HFD), and then administered orally with a *L. Plantarum GB104* strain, were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or a *L. Plantarum GB104* strain was orally administered daily at 5×10⁹ CFU per mouse.

After the administration of each test substance was completed, brown adipose tissue was separated from the mice in each group by autopsy. The separated brown adipose tissue was fixed with 10% neutral buffered formalin. Then, paraffin sections were prepared, stained with H&E, and then the size of the cells was observed.

The result showed that the size of the adipocytes in the negative and positive control groups mice fed with high fat diet was increased compared to the normal group, while the size of the adipocytes in the experimental group to which the *L. Plantarum GB104* strain was administered orally was significantly decreased (FIG. 5). Based on this, it was confirmed that the *L. Plantarum GB104* strain effectively inhibits fat accumulation in brown adipose tissue.

### Example 4. Confirmation of fat accumulation inhibitory effect of L. Plantarum GB104 strain in liver tissue

Using a mouse model, fat accumulation inhibitory effect of a *L. Plantarum GB104* strain in liver tissue was confirmed. Specifically, C57BL/6 mice which were fed with 60% (HFD), and then administered orally with the *L. Plantarum GB104* strain, were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or *a L. Plantarum GB104* strain was orally administered daily at 5×10⁹ CFU per mouse.

After the administration of each test substance was completed, the liver was extracted by autopsy, and the weight was measured to compare and evaluate the weight between groups. After the extracted liver was fixed with 10% buffered formalin, paraffin sections were prepared, stained with H&E, and then the size of the cells was observed.

The result showed that the liver weight of the negative and positive control group mice fed with high fat diet were increased compared to the normal group, while the liver weight in the experimental group mice to which the *L. Plantarum GB104* strain was administered orally were significantly decreased compared to the negative control group (FIG. 6). Furthermore, compared to the normal group, fat accumulation in liver tissue was increased in the negative and positive control group mice fed with high fat diet. However, little fat was accumulated in liver tissue of the experimental group mice to which the *L. Plantarum GB104* strain administered orally, and fat accumulation level was similar to that of the negative control group (FIG. 7). Based on this, it was confirmed that the *L. Plantarum GB104* strain effectively inhibits the liver weight and fat accumulation in liver tissue.

### Example 5. Confirmation of fasting blood glucose-lowering and glucose tolerance improvement effects of L. Plantarum GB104 strain

Glucose tolerance test (GTT) was performed to confirm glucose tolerance improvement effect of a *L. Plantarum GB104* strain. First, C57BL/6 mice which were fed with 60% (HFD), and then administered orally with a *L. Plantarum GB104* strain, were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or a *L. Plantarum GB104* strain was orally administered daily at 5×10⁹ CFU per mouse.

To perform glucose tolerance test, mice were fasted for 16 hours or more, and then a glucose solution was injected intraperitoneally at a dose of 1 g/kg. Then, blood was collected from the tail vein of the mice after 0, 30, 60, 90, and 120 minutes, and blood glucose was measured using a blood glucose meter. In this case, blood glucose at 0 minutes refers to fasting blood glucose.

The result showed that the fasting blood glucose level was significantly increased in the negative and positive control group mice fed with high fat diet, compared to the normal group. Meanwhile, the fasting blood glucose level of the experimental group mice to which the *L. Plantarum GB104* strain was administered orally was significantly decreased compared to the negative control group (FIG. 8).

In addition, it was confirmed that the blood glucose level after administration of glucose in the negative and positive control group mice fed with high fat diet was higher than that of the normal group, while the blood glucose level of the experimental group mice to which the *L. Plantarum GB104* strain was administered orally was significantly reduced compared to the negative control group (FIG. 9). Based on this, it was confirmed that the *L. Plantarum GB104* strain exhibits fasting blood glucose-lowering and glucose tolerance improvement effects.

### Example 6. Confirmation of insulin resistance ameliorating effect of L. Plantarum GB104 strain

Insulin tolerance test (ITT) was performed to confirm insulin resistance ameliorating effect of a *L. Plantarum GB104* strain. First, C57BL/6 mice which were fed with a 60% (HFD), and then administered orally with a *L. Plantarum GB104* strain, were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or *L. Plantarum GB104* strain was orally administered daily at 5×10⁹ CFU per mouse.

To perform insulin tolerance test, mice were fasted for 4.5 hours, and then an insulin solution was administered intraperitoneally at a dose of 1 U/kg. Blood was collected from the tail vein of the mice after 0, 30, 60, 90 and 120 minutes, and then blood glucose was measured with a blood glucose meter.

The result confirmed that the blood glucose level after administration of insulin in the negative and positive control group mice fed with high fat diet was higher than that of the normal group, while the blood glucose level of the experimental group mice to which *the L. Plantarum GB104* strain was administered orally was significantly decreased compared to the negative control group (FIG. 10). Based on this, it was confirmed that the *L. Plantarum GB104* strain effectively ameliorates insulin resistance by increasing insulin sensitivity.

### Example 7. Confirmation of GLP-1 secretion-promoting effect of L. Plantarum GB104 strain

GLP-1 regulatory effect caused by administration of a *L. Plantarum GB104* strain was confirm using a mouse model. Specifically, C57BL/6 mice which were fed with 60% (HFD), and then administered orally with a *L*. *Plantarum GB104* strain, were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or a *L. Plantarum GB104* strain was orally administered daily at 5×10⁹ CFU per mouse.

Serum was isolated from blood of the mice in each group, and a concentration of GLP-1 in serum was analyzed by using a Bio-plex Pro Mouse Diabetes 8-plex assay kit (Bio-rad, Hercules, CA, USA). The sample analysis procedure was performed by referring to the protocol in the Kit.

The result showed that the concentration of GLP-1 in serum of the negative and positive control group mice fed with high fat diet was lower or similar compared to the normal group, while the concentration of GLP-1 of the experimental group mice to which the *L. Plantarum GB104* strain was administered orally was significantly higher than the negative control group (FIG. 11). Based on this, effect of *L. Plantarum GB104* strain to increase the concentration of GLP-1 in serum was confirmed.

### <Accession Number>

Name of Depository : Korean Collection for Type Cultures (KCTC), Korea Research Institute of Bioscience and Biotechnology
Accession Number : KCTC14107BP
Date of Deposit: 20200114.

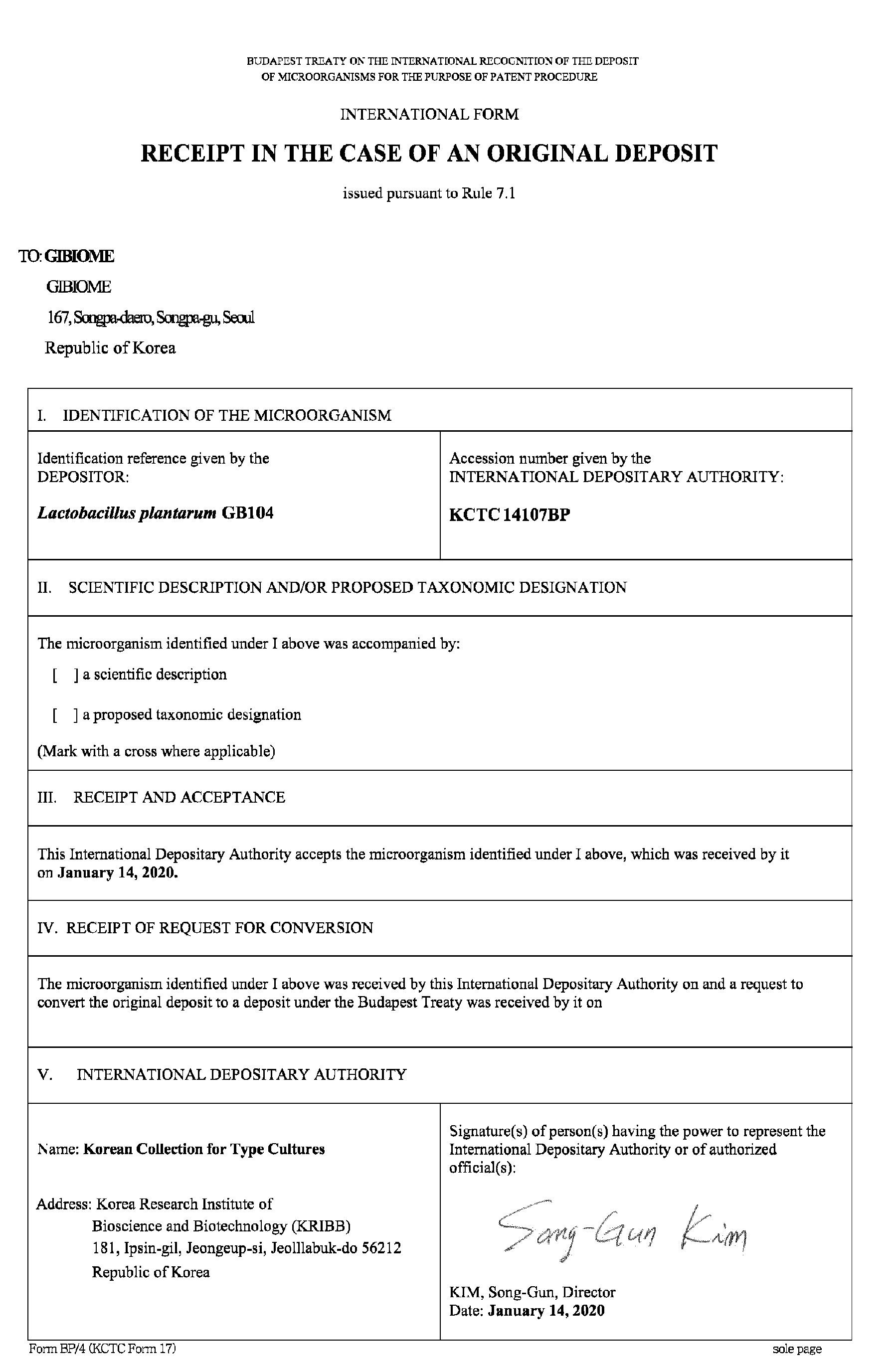
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: GIBIOME
GIBIOME
167, Songpa-daero, Songpa-gu, Seoul
Republic of Korea

I. IDENTIFICATION OF THE MICROORGANISM
Identification reference given by the
DEPOSITOR:

Lactobacillus plantarum GB104 | Accession number given by the
INTERNATIONAL DEPOSITARY AUTHORITY:

KCTC 14107BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on January 14, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):


KIM, Song-Gun, Director
Date: January 14, 2020
Form BP/4 (KCTC Form 17) sole page

## Claims

1. A *Lactobacillus Plantarum* strain (Accession No. KCTC 14107BP).

2. A pharmaceutical composition for preventing or treating a metabolic disease comprising, as an active ingredient, a *Lactobacillus Plantarum* strain (Accession No. KCTC 14107BP) or a culture thereof.

3. The pharmaceutical composition for preventing or treating a metabolic disease of claim 2, wherein the metabolic disease is selected from the group consisting of hypertension, arteriosclerosis, hyperlipidemia, non-alcoholic fatty liver disease, hyperinsulinemia, type 2diabetes, and insulin resistance syndrome.

4. The pharmaceutical composition for preventing or treating a metabolic disease of claim 2, wherein the strain inhibits fat accumulation in white adipose tissue.

5. The pharmaceutical composition for preventing or treating a metabolic disease of claim 2, wherein the strain reduces fat accumulation in brown adipose tissue.

6. The pharmaceutical composition for preventing or treating a metabolic disease of claim 2, wherein the strain inhibits fat accumulation in liver tissue.

7. The pharmaceutical composition for preventing or treating a metabolic disease of claim 2, wherein the strain reduces a blood glucose level.

8. The pharmaceutical composition for preventing or treating a metabolic disease of claim 2, wherein the strain reduces insulin resistance.

9. The pharmaceutical composition for preventing or treating a metabolic disease of claim 2, wherein the strain regulates a concentration of metabolic hormones in blood.

10. The pharmaceutical composition for preventing or treating a metabolic disease of claim 9, wherein the metabolic hormone is GLP-1.

11. A food composition for preventing or inhibiting a metabolic disease comprising a *Lactobacillus Plantarum* strain (Accession No. KCTC 14107BP) or a culture thereof.

12. A feed composition for preventing or inhibiting a metabolic disease comprising a *Lactobacillus Plantarum* strain (Accession No. KCTC 14107BP) or a culture thereof.

13. A method of preventing or treating a metabolic disease comprising:
administering a *Lactobacillus Plantarum* strain (Accession No. KCTC 14107BP) or a culture thereof to a subject.
